# EUROPEAN PATENT SPECIFICATION

(11) **EP 2 410 934 B1**
(45) Date of publication and mention of the grant of the patent: **26.08.2015**
(21) Application number: 10713309.2
(22) Date of filing: 23.03.2010
(51) Int. Cl.: A61B 17/70

(54) **SPINE FIXATION SYSTEM**
WIRBELSÄULENFIXIERUNGSSYSTEM
Système de fixation de la colonne vertébrale

(30) Priority: 26.03.2009 EP 09004363; 29.04.2009 EP 09005904
(43) Date of publication of application: 01.02.2012
(73) Proprietor: SPONTECH SPINE INTELLIGENCE GROUP AG, 70182 Stuttgart (DE)
(72) Inventor: COPF, Franz, 70184 Stuttgart (DE)
(74) Representative: Schwanhäußer, Gernot
(86) International application number: PCT/EP2010/001811
(87) International publication number: WO 2010/108655

(56) References cited:
- EP-A1- 1 210 914
- WO-A2-2006/065666
- US-A1- 2006 036 252
- US-A1- 2006 235 389
- US-A1- 2007 288 004

## Description

### BACKGROUND OF THE INVENTION

### 1. Field of the Invention

The present invention relates to a spine fixation system for the surgical treatment of spinal disorders which may require correction, stabilization, adjustment or fixation of the spinal column.

### 2. Description of Related Art

Various types of spinal column disorders are known and include scoliosis (abnormal curvature or rotation of vertebrae relative to the plane of the spine), kyphosis (abnormal backward curvature of the spine) and spondylolisthesis (forward displacement of a lumber vertebra), all of which involve a "misalignment" of the spinal column. Patients who suffer from such conditions usually experience extreme, debilitating pain and physical deformity due to the condition. In severe cases treatments for these conditions have used a technique known as fusion with spinal fixation which results in the mechanical immobilization of areas of the spine and the eventual fusion of the vertebrae in the regions treated. In less severe cases treatment comprises decompression of the affected nerves and fusion of the vertebrae involved.

Fusion, however, is not usually successful unless the vertebrae are also fixed for a time period by a mechanical device installed internally during surgery. This allows the fused bone time to heal. Numerous mechanical systems have been proposed for this purpose. Screw and rod systems and screw and plate systems are commonly used to this purpose. The former system typically uses a rigid rod secured to the spine by screws inserted in the pedicles for holding the rod. The rod may be bent to the desired configuration, and this both manipulates and holds the vertebrae in that same configuration until the fusion process can permanently accomplish the same thing.

Since each patient has his or her own spinal characteristics or anatomy, including bone shape and bone density, the exact location of the pedicle screws and the other parts of the spine fixation system is determined while the patient is on the operating table. Bending the rod consumes operating time, because contouring the rod to correspond to the three-dimensional configuration of the spine can be extremely difficult and can lead to mistakes. Even if the three-dimensional anatomic orientation of the pedicle is ascertained prior to surgery, there is often a need to re-orient these screws in the pedicles and to readjust their depth of insertion. This often requires complete screw removal, particularly if the spinal deformation was not exactly as anticipated or if the space available for the screw was insufficient. Removing and replacing screws jeopardizes the fragile bone structure of the pedicle around the screw holes, and it also consumes additional surgical time.

It has therefore been proposed to design the connectors, which connect the pedicle screws and the rod, such that the rod can be moved with regard to the pedicle screw with one or more degrees of freedom. This reduces the need to re-orient or readjust pedicle screws and requires less effort to bend the rod.

US 5,545,166 A describes a spine fixation system in which each connector comprises a threaded bolt that is pivotably attached to the head of a pedicle screw. A pivot block is threaded onto the bolt so that the pivot block can move up and down the bolt. A clamp block receiving the rod is pivotably attached to the pivot block.

US 5,254,118 A describes a spine fixation system in which each connector comprises a tulip-like seat member which receives the rod and has a bore through which a pivot attached to a main portion of the connector extends. The seat member can be clamped in various angular positions with respect to this pivot, which is arranged perpendicular to the pedicle screw or forms a small angle therewith. The seat member can also be moved along the pivot which offers an additional degree of freedom. The main body of the connector can be fixed at various axial positions on the pedicle screw. The rod can therefore be rotated around a rotational axis which extends perpendicular to (or forms a small angle with) the longitudinal axis of the pedicle screw.

US 2006/0235389 A1 discloses a spine fixation system comprising pedicle screws having a screw shaft and a ball-shaped head portion. A neck portion of the screws includes a pair of 90° turns so that the head portion is offset the shaft. The connector consists of a seat member arranged on the head portion so that it can be polyaxially adjusted with regard to the pedicle screw.

US 2007/0288004 A1 discloses a spine fixation system that also comprises pedicle screws having a screw shaft and a ball-shaped head portion. Here, however, the head portion is centered with regard to the shaft. The connector comprises a head member arranged on the head portion of the screw so that it can be polyaxially adjusted with regard to the pedicle screw. The head member has an arm with a bushing in which a tulip-like seat member is pivotably received. The seat member is thus allowed to rotate around an axis that runs parallel to the longitudinal axis of the pedicle screw. The seat member is fixed in the bushing by screwing in a lid from the opposite side. Thus the seat member cannot be fixed on the arm once the head member is mounted on the head portion of the pedicle screw.

In the aforementioned prior art spine fixation systems the longitudinal axis of the rod is always arranged eccentrically with regard to the longitudinal axis of the pedicle screw. Other types of connectors do not have such an eccentric arrangement. Instead, the longitudinal axis of the rod and the pedicle screw cross each other or are spaced apart by a very small distance, only. In order to still provide a minimum degree of flexibility, the connector may enable polyaxial movements of the rod with regard to the pedicle screw during the surgery. Such prior art spine fixation systems are disclosed, for example, in US 2006/0172056 A1, US 2008/0243193 A1 and EP 1 295 566 A1.

### SUMMARY OF THE INVENTION

It is an object of the present invention to provide a spine fixation system which offers an improved flexibility so that the surgeon can easily adjust the rod to pedicle screws or other types of fasteners.

This object is achieved, according to the present invention, by providing a spine fixation system which comprises a rod which is configured to extend over a portion of the spine. The rod may be straight or curved, and it may be rigid or ductile so that it can be bent into a desired configuration. The system further comprises a plurality of fasteners each having a longitudinal axis and being configured to be secured to a vertebra to be treated. A plurality of connectors is provided, wherein each connector is connected to, or is capable of being connected to, one of the fasteners. Furthermore each connector has a seat member, which may be formed by a tulip, for receiving and fixing the rod. The seat member is capable of being fixed in different rotational positions with regard to a rotational axis that extends, or is capable of being positioned such that it extends, parallel to but not coinciding with the longitudinal axis of the fastener to which the respective connector is connected. The seat member is configured such that the rod is allowed, when received in the seat member, to swivel over the fastener when the seat member rotates around the rotational axis. Furthermore, the seat member comprises a head member which is connected to, or is capable of being connected to, one of the fasteners. The head member supports the seat member so as to enable at least rotational movement of the seat member with regard to the head member before the position of the seat member is fixed. The head member is capable of being fixed to the fastener, to which the respective connector is connected, in different rotational positions with regard to the longitudinal axis of this fastener.

Providing an eccentric rotational axis for the seat member, i.e. a rotational axis that runs parallel to but does not coincide with the longitudinal axis of the fastener, provides a superior flexibility. The head member is also able to rotate around the longitudinal axis of the fastener. Then the connector can be rotated around the fastener without a need to rotate the fastener, too. Rotating the fastener is impossible if it is cemented into a bore in the respective vertebra. But also if the fastener is a screw, such rotations are undesirable because rotating the screw in the vertebra changes not only its axial position, but also compromises the solidity of the screw connection.

Since there are two parallel rotational axes available, any rod, which extends (at least almost) arbitrarily through a circular area centered with respect to the fastener, can be easily fixed. The larger the distance between the two rotational axes is, the larger is the area within which the rod may be received by the seat member.

This is particularly useful if the fasteners are configured to be secured to a pedicle of the vertebra to be treated. For various reasons it is sometimes inevitable to secure the fasteners in the pedicles such that their free ends extending above the pedicles are not parallel and do not lie along a straight line. In this case the ability to position the seat member using the two degrees of freedom (i.e. rotations by the two parallel rotational axes) makes it possible to connect the fasteners to the rod even under very difficult spatial conditions.

According to a preferred embodiment the rotational axis is, for enabling the seat member to be polyaxially adjusted, capable of being fixed in different tilting positions at least within a cone of tilting angles. Such a polyaxial adjustability further increases the range of positions into which the seat member can be brought before the components of the spine fixation system are fixed. For example, the rod is now allowed to perform, together with the seat member, variable tilting movements. Without a polyaxial adjustability such tilting movements of the rod are only possible if the rod tilts within the (fixed) seat member. This, however, will usually result in small contact areas, for example lines or even single points, between the seat member and the rod which is generally undesirable. A polyaxial adjustability furthermore enables the rotational axis to be tilted such that the rod changes its height with respect to the fastener such that there is no need to readjust the fastener or the connector attached to it.

The axis of symmetry of the cone is arbitrary to the extent that the cone at least contains a direction which is parallel to the longitudinal axis of the fastener. In one embodiment the axis of symmetry of the cone extends parallel to, but does not coincide with, the longitudinal axis of the fastener to which the respective connector is connected. In other words, if the rotational axis is not tilted within the cone, it automatically extends parallel to, but does not coincide with, the longitudinal axis of the fastener.

In one embodiment each connector is configured such that it can be connected to the respective fastener after the fastener has been secured to the vertebra to be treated. This facilitates the implant of the fastener into the vertebra because no connector obstructs the way for inserting a suitable tool. Once the fastener is implanted, the connector is attached to the fastener and finally fixed to the fastener with the help of suitable fixing means, for example a fixing screw.

The seat member may have a recess for receiving the rod. This recess may, in its cross-section, be U-shaped which results in a tulip-like seat member. At the open end of the U-shaped recess the rod can be easily inserted. The recess may still enable axial displacement of the rod within the recess for performing final adjustments of the rod within a row of seat members arranged one behind the other along the human spine.

In another embodiment each connector is configured such that the rod is allowed, when received in the seat member of the connector, to swivel over the fastener when the seat member rotates around the rotational axis. In other words, neither the fastener nor any part of the connector obstructs a free 360° degree rotation of the rod received in the seat member.

The connector may comprise a clamp mechanism for fixing the rod to the seat member. Such a clamp mechanism may comprise a fixing screw, for example.

In a preferred embodiment the clamp mechanism is configured such that operation of the clamp mechanism by a user fixes the rod to the seat member and simultaneously fixes the seat member to the head member. Then a surgeon may first adjust the connector such that the rod is fully received in the seat member, and he may then fix the rod to the seat member and the seat member to the head member in one go by a single operation of the clamp mechanism. The effect of arresting two degrees of freedom in one go may be achieved if the pressure exerted by a clamp screw or a similar element is passed on, preferably via the rod itself, to a second clamping element which serves to fix the seat member to the head member.

Since the head member is allowed to rotate around the longitudinal axis of the fastener without also rotating the fastener itself, it is even possible to fix, by operation of the clamp mechanism, not only the rod to the seat member and simultaneously the seat member to the head member, but also the head member to the fastener. This may be achieved by passing the force exerted by a clamp screw or a similar element not only to the head member, but also to the fastener, for example by using an appropriate leverage.

According to a still further embodiment the head member comprises a first head portion, which is fixed to the fastener or is capable of being fixed to it, and a second head portion, which supports the seat member. The second head portion is capable of being fixed to the first head portion in different rotational positions with regard to a further rotational axis that is arranged non-parallel, and in particular perpendicularly, to the longitudinal axis of the fastener. Such an additional degree of rotational freedom further enhances the flexibility of the connector so that the surgeon may more easily adjust the rod to the pedicle screws or other types of fasteners. If the rotational axis, around which the seat member is allowed to rotate before it is fixed, extends parallel to the further rotational axis, around which the first head portion is capable to rotate before it is fixed, the height of the rod may be adjusted without changing the rod's lateral position.

The present invention also describes a method of implanting a spine fixation system. The method comprises the following steps:
a) securing fasteners to at least three different vertebrae to be treated;
b) connecting a rod to the fasteners using connectors that enable relative movements between the fasteners and the rod before the rod is rigidly fixed to the fasteners;
c) rigidly fixing the rod to the fasteners secured in two of the at least three vertebrae;
d) repositioning at least one of the fasteners that has not yet been rigidly fixed to the rod, thereby moving the fasteners with respect to the rod;
e) rigidly fixing the rod to the repositioned fastener.

Prior art spine fixation systems do not, or do only to a very limited extent, make it possible to reposition one or more vertebrae. This is because the rods are contoured in the prior art systems such that they follow the contour of the fasteners, and not the other way round. When fasteners using connectors that enable relative movements between the fasteners and the rod before the rod is rigidly fixed to the fasteners are used, the fasteners (and thus the vertebrae) can be repositioned with respect to a stationary rod.

### BRIEF DESCRIPTION OF THE DRAWINGS

Various features and advantages of the present invention may be more readily understood with reference to the following detailed description taken in conjunction with the accompanying drawing in which:
- FIG. 1: is a perspective view on a spine fixation system in accordance with the present invention implanted in a segment of the human spine;
- FIG. 2: is top view on the spine fixation system shown in FIG. 1;
- FIG. 3: is a perspective view of a connector mounted on a pedicle screw of the spine fixation system shown in FIGS. 1 and 2;
- FIG. 4: is a side view of the components shown in FIG. 3;
- FIG. 5a: is a sectional view through a seat member of the connector in a first position;
- FIG. 5b: is a sectional view similar to FIG. 5a, but for a different position of the seat member;
- FIG. 6a: is a perspective view of the connector and a top portion of the pedicle screw, with the seat member in a first position;
- FIG. 6b: is a perspective view similar to FIG. 6a, but with the seat member in a different position;
- FIG. 7: is a sectional view through a head portion of the connector;
- FIG. 8: is a flow diagram illustrating a surgical method of implanting the spine fixation system;
- FIG. 9: is a perspective view of a connector similar to FIGS. 6a and 6b according to another embodiment in which the connector has an additional degree of rotational freedom;
- FIG. 10: is a cross-section similar to FIGS. 5a and 5b according to an embodiment in which the rod and the tulip can be fixed simultaneously by a clamp screw;
- FIG. 11: is a perspective partial section of the tulip shown in FIG. 10;
- FIG. 12: is in an exploded view of the tulip and the portion of the projection shown in FIG. 10;
- FIG. 13: is a cross-section through the tulip and a portion of the projection similar to FIG. 10 according to a still further embodiment in which the rod, the tulip and the head member can be fixed simultaneously by a clamp screw.

### DESCRIPTION OF PREFERRED EMBODIMENTS

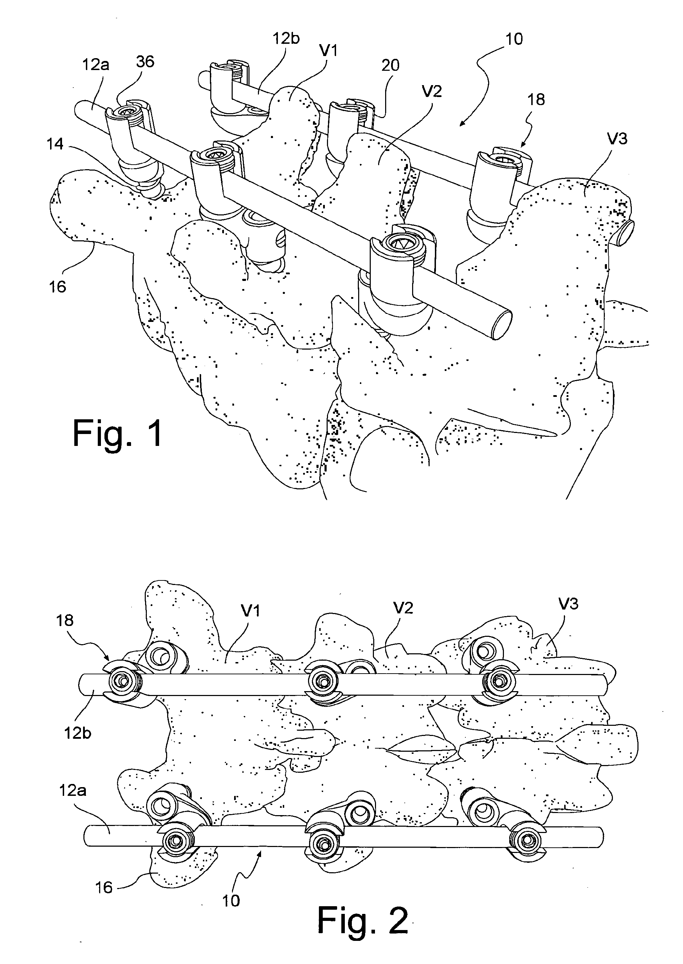

A spine fixation system in accordance with the present invention is shown in the perspective view of FIG. 1 and the top view of FIG. 2 after having been implanted in a segment of a human spine. This segment comprises three vertebrae V1, V2, V3 which are arranged one behind the other. For the sake of simplicity the vertebrae adjacent to this segment are not shown in FIGS. 1 and 2.

The spine fixation system, which is denoted in its entirety by 10, comprises two rods 12a, 12b which extend on either side of the spine segment along a longitudinal direction of the spine. In this embodiment the rods 12a, 12b are rigid straight elements made of a suitable metal, for example titanium. In other embodiments the rods 12a, 12b have a curved shape. It is further envisaged to use ductile rods or rods having other than circular cross-sections, for example an oval or polygonal cross-section.

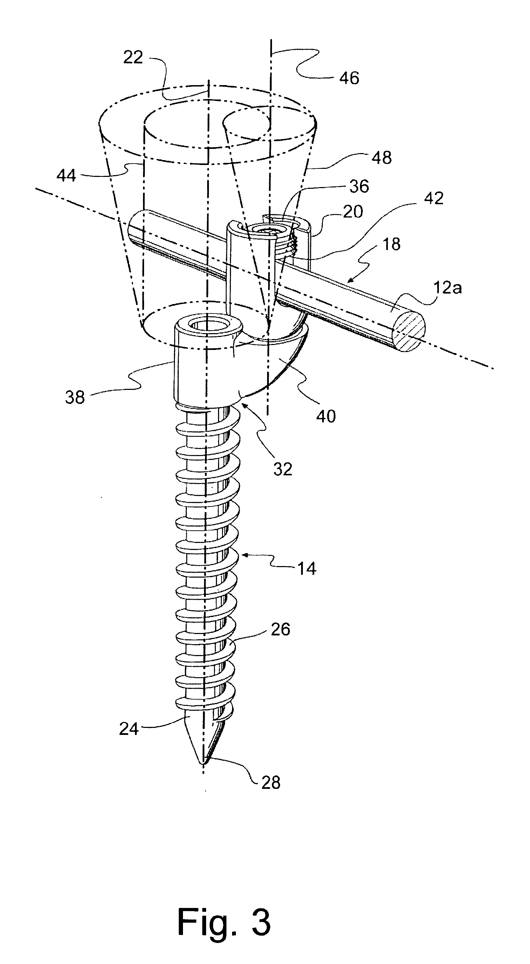

The rods 12a, 12b are fastened to the vertebrae V1, V2 and V3 with the help of pedicle screws 14, which are secured in the pedicles 16 of the vertebrae V1, V2 and V3, and connectors 18 that connect the pedicle screws 14 to the rods 12a, 12b. As it will become apparent from the following description of the connectors 18 with reference to FIGS. 3 to 7, the connectors 18 each have a seat member, in this embodiment formed by a tulip 20, which has various degrees of freedom so that it can be positioned in many different ways. This makes it possible to connect the straight rods 12a, 12b to the pedicle screws 14 although the latter are arranged neither parallel to each other nor along a straight line, as can best be seen in the top view of FIG. 2.

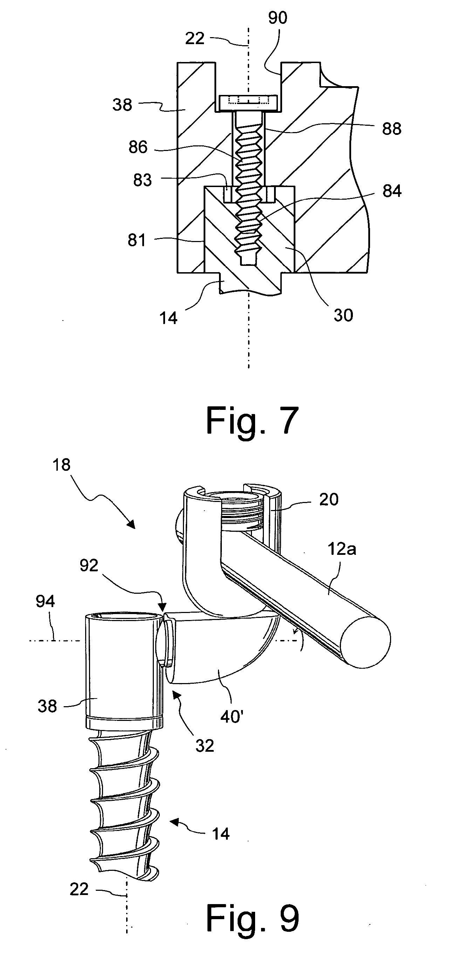

FIG. 3 shows one of the pedicle screws 14 and one of the connectors 18 in a perspective view. The pedicle screw 14 has a longitudinal axis 22 and a cylindrical portion 24 supporting an external thread 26. At one end the pedicle screw 14 has a conical tip 28 and at its opposite end a screw head 30 which can, because it is covered by the connector 18, only be seen in the cross-section of FIG. 7 that will be explained further below.

The pedicle screw 14 is configured with regard to its length, diameter and the external thread 26 such that it can be screwed into the pedicle 16 of any of the vertebrae V1 to V3. However, not only screws but other types of fasteners may be used to this end. Such fasteners include, but are not limited to, bolts which have ridges on their outer surfaces and can be cemented into cylindrical bores drilled in the pedicles 16 of the vertebrae V1 to V3.

The connector 18 comprises a head member 32, a seat member formed by the tulip 20, and a clamp screw 36. The head member 32 includes a cylindrical portion 38, which is mounted on the screw head 30 of the pedicle screw 14, and a projection 40 which is, in the embodiment shown, formed integrally with the cylindrical portion 38. The tulip 20 has a recess 42 which is configured to receive one of the rods 12a or 12b. An upper portion of the recess 42 has an internal thread which is adapted to an external thread of the clamp screw 36. By screwing the clamp screw 36 into the recess 42, it is thus possible to secure the rod 12a in the recess 42. As a matter of course, other types of clamp mechanism may be used instead.

The connector 18 has various degrees of freedom which will be explained now with reference to FIG. 3. As a matter of course, these degrees of freedom are only available during the surgery, i.e. when the surgeon has implanted the pedicle screws 14 in the pedicles 16 and has now to connect the pedicle screws 14 to the rods 12a, 12b. Once the pedicle screws 14 are connected to the rods 12a, 12b and the vertebrae V1 to V3 are in the desired position, all movable elements of the spine fixation system 10 will be fixed by the surgeon so that no degrees of freedom are available any more. However, for being able to quickly connect the pedicle screws 14 to the rods 12a, 12b without a need to readjust the pedicle screws 14 or to bend the rods 12a, 12b, it is crucial that the surgeon is able to quickly accomplish these connections whilst still being able to arrange the vertebrae V1 to V3 if desired.

In the embodiment shown the connector 18 is capable of being fixed in different rotational positions with regard to the longitudinal axis 22. This ability to rotate around the longitudinal axis 22 provides a first degree of rotational freedom. While the cylindrical portion 38 of the connector 18 remains centered with respect to the longitudinal axis 22 during such rotations, the projection 40 supporting the tulip 20 swivels around the longitudinal axis 22, as is indicated by a cylinder 44 in FIG. 3.

Furthermore, the tulip 20 is capable of being fixed in different rotational positions with regard to a rotational axis 46 which does not coincide to the longitudinal axis 22. At least if the rotational axis 46 runs parallel to the longitudinal axis 22 of the pedicle screw 14, as is indicated in FIG. 3, the rod 12a is allowed to swivel over the pedicle screw 14 and the head portion 32 of the connector 18 during such rotations of the tulip 20. In other words, the rod 12a is allowed to perform a rotation by 360° which is not blocked by any part of the pedicle screw 14 or the connector 18. This property can also be seen in FIG. 2 in which the connector 18, which connects the rod 12b to the vertebra V2, is in a configuration in which the rod 12b extends over the pedicle screw 14.

In the embodiment shown a third degree of freedom is provided in that the rotational axis 46, around which the tulip 20 is allowed to rotate, is not fixed, but can be polyaxially adjusted within a cone of tilting angles which is denoted in FIG. 3 by 48. Thus the tulip 20 can not only be rotated, but also tilted into various directions. In this embodiment the cone 48 has an axis of symmetry which extends parallel to, but does not coincide with, the longitudinal axis 22 of the pedicle screw 14. If the tulip 20 is not tilted within the cone 48, its rotational axis 46 therefore coincides with the axis of symmetry of the cone 48 and thus also runs parallel to the longitudinal axis 22.

In other embodiments, the axis of the symmetry of the cone 48 does not run parallel to the longitudinal axis 22, but forms an angle therewith, which is significantly smaller than 90°, preferably smaller than 45°. This may be advantageous at least for certain vertebrae of the human spine where there is insufficient space for guiding the rods 12a, 12b closely to the pedicle screw 14. In still other embodiments the position of the rotational axis 46 is permanently fixed and cannot be adjusted at all (i.e. the cone angle is zero).

Referring back to the embodiment shown in FIG. 3, the surgeon is, by combining the aforementioned three degrees of freedom, able to position the tulip 20 easily to a desired position such that it can receive the rod 12a.

If the rod 12a shall also be adjusted in height, i.e. along the longitudinal axis 22 of the pedicle screw 14, it may be envisaged to screw in or out the pedicle screw 14 such that it moves, together with the connector 18, along its longitudinal axis 22. Alternatively, the connector 18 may be attached to the pedicle screw 14 in a manner that enables an adjustment along the longitudinal axis 22. This may be accomplished, for example, by screwing the cylindrical portion 38 of the connector 18 onto the head 30 of the pedicle screw 14 and to provide an additional sleeve so that the projection 40 can be rotated around the longitudinal axis 22 without affecting the connection between the connector 18 and the pedicle screw 14.

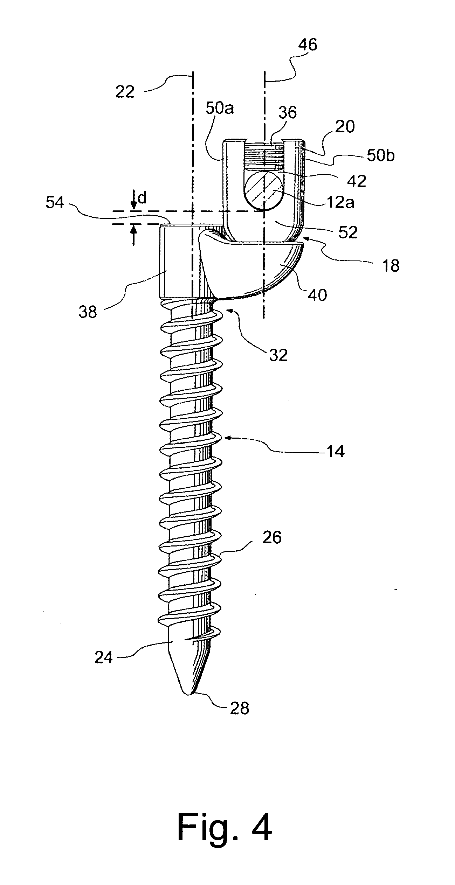

FIG. 4 is a side view of the pedicle screw 14 with the connector 18 mounted on top of it. In this side view it can be seen that the recess 42 in the tulip 20 is defined by two limbs 50a, 50b and a base portion 52 connecting the limbs 50a, 50b. The rod 12a rests on this base portion 52 and thus defines the position of the rod 12a along the longitudinal axis 22. The upper end 54 of the cylindrical portion 38 of the connector 18 is spaced apart by a distance d from the upper end of the base portion 52. Therefore the rod 12a is allowed to swivel over the pedicle screw 14 and the cylindrical portion 38 of the connector 18 when the tulip 20 rotates around the rotational axis 46. This is even possible if the rotational axis 46 is tilted by a small angle within the cone 48.

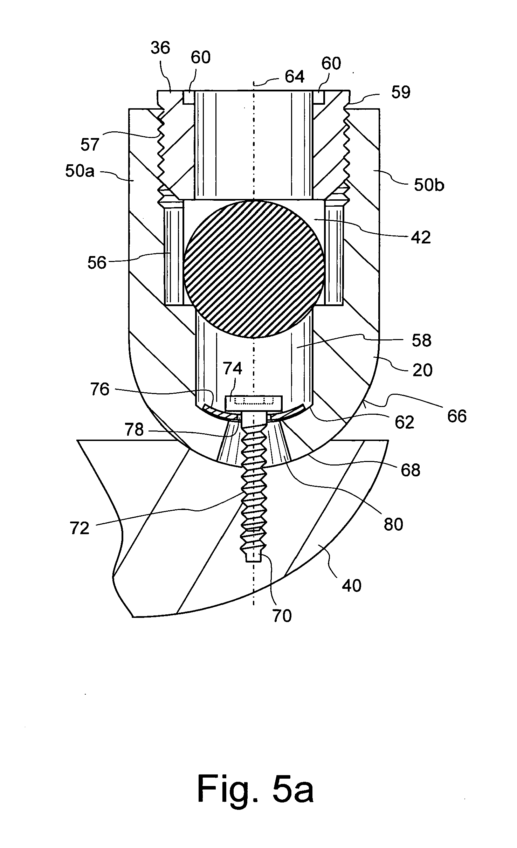

FIG. 5a is a cross-section through the tulip 20 and a portion of the projection 40 of the connector 18. In this cross-section it can be seen that the central portion of the tulip 20 has a stepped bore, with an upper bore portion 56 having a larger diameter and a lower bore portion 58 having a smaller diameter. An upper half of the upper bore portion 56 is provided with an internal thread 57 which is adapted to an external thread 59 of the clamp screw 36. The clamp screw 36 is provided at its upper end with indentations 60 adapted to receive a tip of a suitable screw driver.

A ground 62 of the lower bore portion 58 is concavely curved, with a center of curvature being arranged on the axis of symmetry 64 of the tulip 20. The lower portion of the tulip 20 has a convex surface 66 and is received in a complementary concave recess 68 formed in the projection 40. The convex surface 66 and the concave recess 68 have centers of curvature which coincide with the center of curvature of the ground 62 of the lower bore portion 58.

The projection 40 further comprises a threaded bore 70 in which a first fixing screw 72 is screwed. A head 74 of the fist fixing screw 72 rests on a curved washer 76 whose center of curvature also coincides with the center of curvature of the ground 62. The washer 76 has a central aperture 78 through which the bolt of the first fixing screw 72 extends.

The ground of the tulip 20 is provided with a ground opening 80 which has, in the embodiment shown, the shape of a cone section. The outer diameter of the washer 76 is determined such that it sufficiently extends over the upper diameter of the ground opening 80, but is still significantly smaller than the diameter of the lower bore portion 58.

If the first fixing screw 72 is not tightened, the tulip 20 is allowed to rotate around its axis of symmetry 64. Furthermore, the tulip 20 as a whole, and thus also the rotational axis 46 coinciding with the axis of symmetry 64, can be tilted.

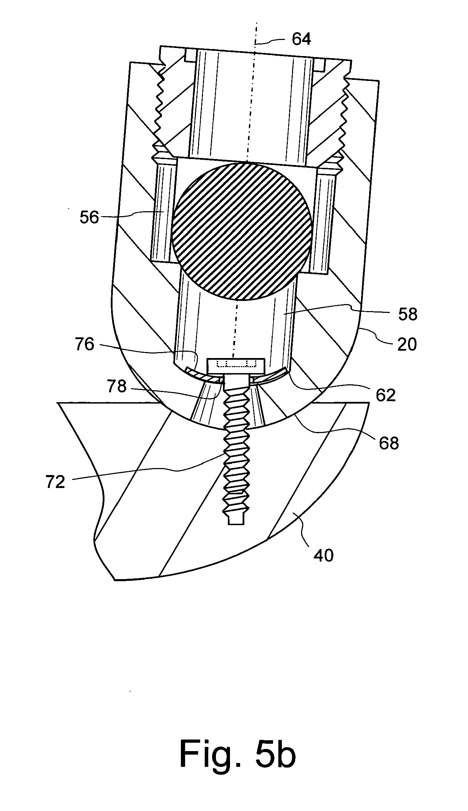

This is shown in FIG. 5b. There it can be seen that the washer 76 has slid along the ground 62 of the lower bore portion 58. The maximum tilt angle, i.e. the opening angle of the cone 48, is determined by the ratio of the diameters of the ground 62 and the washer 76. Also in the tilted position the tulip 20 can still rotate around its axis of symmetry 64 as long the first fixing screw 72 has not yet been tightened.

This design therefore enables a polyaxial adjustment of the tulip 20 with respect to the projection 40 of the connector 18. After the tulip 20 is brought approximately in a rotational and tilting position that is required to receive the rod 12a, the latter may be inserted from above in the recess 44. This will often result in additional small movements of the tulip 20. Then the rod 12a is carefully removed and the first fixing screw 72 is tightened. After tightening the first fixing screw 72, the tulip 20 is fixed with respect to the projection 40 and thus cannot perform any movements. Then the rod 12a is inserted again and secured with the help of the clamp screw 36.

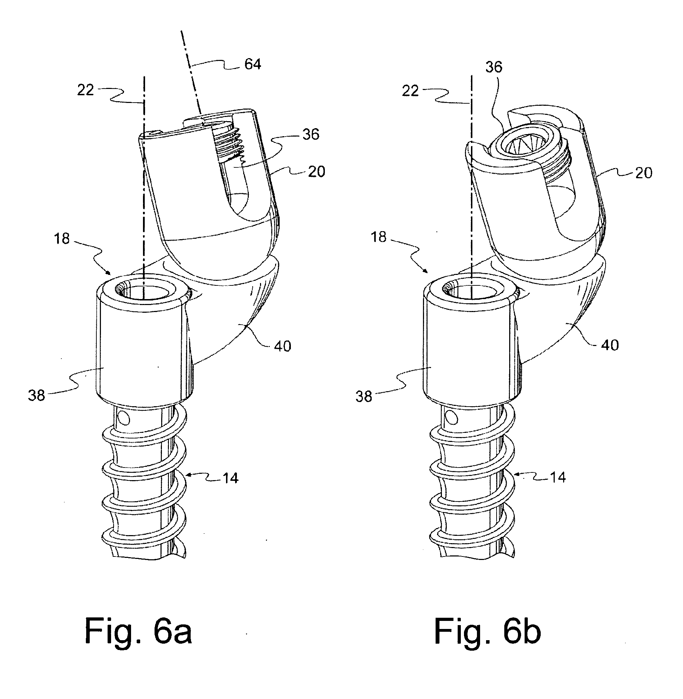

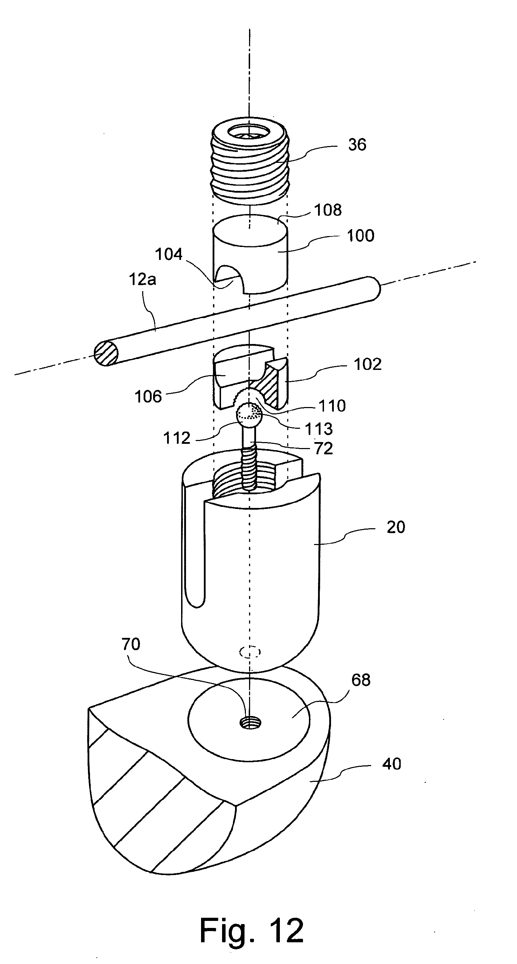

FIGS. 6a and 6b are perspective views showing the connector 18 and an upper portion of the pedicle screw 14 for two different positions of the tulip 20. For the sake of simplicity the rod 12a is not shown in FIGS. 12a and 12b. The position shown in FIG. 6b is obtained from the position shown in FIG. 6a by first rotating the connector 18 on the pedicle screw 14 by a few degrees in the counterclockwise direction around the longitudinal axis 22. Furthermore, the tulip 20 is rotated in the clockwise direction by some degrees around its axis of symmetry 64. Furthermore, the tulip 20 is tilted, using the polyaxial adjustability, towards the longitudinal axis 22 of the pedicle screw 14.

FIG. 7 is a cross-section through the cylindrical portion 38 of the connector 18 and the screw head 30 of the pedicle screw 14. The cylindrical portion 38 has a lower bore 81 which has a slightly larger diameter as the pedicle screw head 30 such that it can rotate on the screw head 30. The screw head 30 has on its top a hexagon socket 83 and is provided with a threaded bore 84 for receiving the lower end of a second fixing screw 86. The threaded bore 84 extends as a widened bore 88 through the cylindrical portion 38 of the connector 18 and then widens again into an upper bore portion 90. The second fixing screw 86 extends through the bore 88 in the cylindrical portion 38, with its head resting on the ground of the upper bore portion 90.

If the second fixing screw 86 is not tightened, the cylindrical portion 38 is allowed to rotate freely around the longitudinal axis 22 of the pedicle screw 14, as has been explained above with reference to FIG. 3. If the connector 18 is in a desired rotational position with regard to the longitudinal axis 22, the second fixing screw 86 is tightened so that no more rotational movements are possible.

### Implant method

In the following it will be described how the spine fixation system 10 may be implanted.

After the surgical site has been prepared, the pedicle screws 14 are secured to the vertebrae V1, V2 and V3 to be treated. To this end the pedicle screws 14 are screwed in the pedicles 16 of the vertebrae V1, V2 and V3 with the help of a hex driver which matches the hexagonal socket 82 provided in the screw head 30 of the pedicle screws 14.

Then the connectors 18 are placed on the screw heads 30 such that the lower bores 81 enclose the screw heads 30. Then the second fixing screws 86 are screwed into the bores 84, but are not yet firmly tightened. Then the tulips 20 of the connectors 18 are adjusted so that their recesses 42 at least approximately lay along a straight line. To this end the connectors 18 may be rotated around the longitudinal axes 22 of the pedicle screws 14, polyaxially tilted and/or rotated around their axes of symmetry 64. Then the rods 12a, 12b are inserted into the recesses 42 of the tulips 20 which results in a final adjustment of the tulip 20. Then the rods 12a, 12b are removed and the first fixing screws 72 and the second fixing screws 86 are tightened. Then the rods 12a, 12b are inserted again and fixedly secured to the tulips 20 with the help of the clamp screws 36.

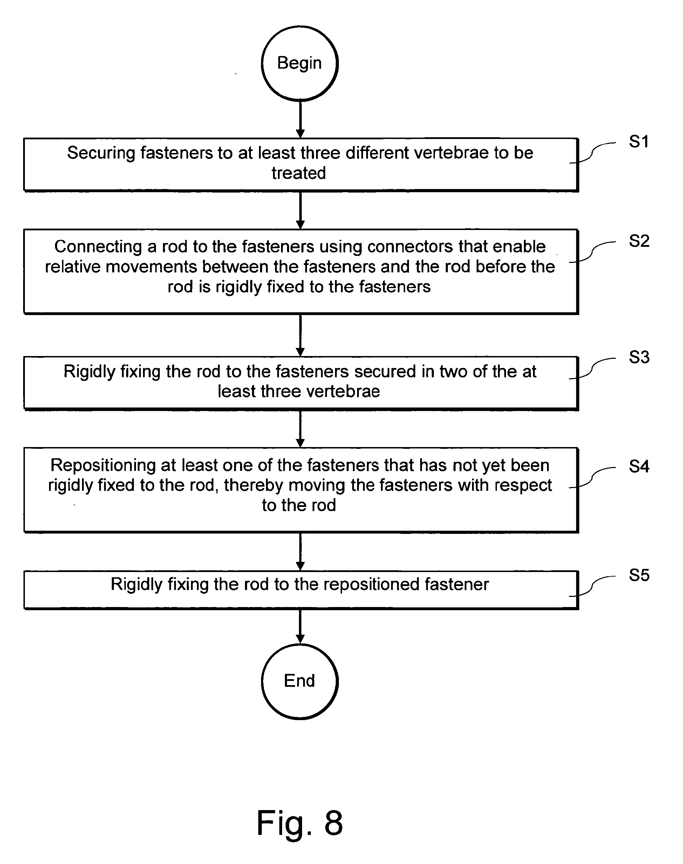

The spine fixation system 10 may also be used to reposition vertebrae during the surgery if more than two vertebrae are to be fixed. In other words, not the rods 12a, 12b will be adjusted with respect to the stationary pedicle screws 14, but the latter will be adjusted with respect to the stationary rods 12a, 12b. This will be explained with reference to the flow diagram shown in FIG. 8.

Also in this case the pedicle screws 14 are secured in a step S1 to the pedicles 16 of the vertebrae V1 to V3. Also the second step S2, namely connecting the rods 12a, 12b to the pedicle screws 14 using the connectors 18 is performed as explained above. Then, however, not all, but at least two vertebrae (not necessarily adjacent ones) are rigidly fixed with the help of the rods 12a, 12b in a step S3. This implies that the first and second fixing screws 72, 84 of the respective connectors 18 are all tightened. Then, in a step S4, those pedicle screws 16, which are attached to the connectors 18 which have not yet been tightened, are repositioned until a desired position of the vertebrae is obtained. Only then the rods 12a, 12b are, in a step S5, rigidly fixed also to the repositioned pedicle screws 14 by tightening the first and second fixing screws 72, 86 of the respective connectors 18.

It is then possible to reposition one or more vertebrae to the rods 12a, 12b that have already been secured to other vertebrae. If the rods 12a, 12b shall remain in place after this repositioning process, other clamping or fixing means may be provided that make it possible to fix the tulips 20 in their final position without removing the rods 12a, 12b from the recesses 42.

### Alternative embodiments

In the following some alternative embodiments will be described with reference to FIGS. 9 to 12.

### a) Further rotational axis

FIG. 9 is a perspective view of a connector 18 according to another embodiment. The connector 18 has basically the same structure as the connector 18 shown in FIGS. 3 to 7. However, in this embodiment the cylindrical portion 38 of the head member 32 is not integrally formed with a protrusion 40. Instead, the head member 32 comprises a separate protrusion portion 40' which also supports the tulip 20, but is connected to the cylindrical portion 38 via a joint 92. The joint 92 is configured such that the protrusion portion 40' is able to rotate, together with the tulip 20, around a further rotational axis 94 with respect to the cylindrical portion 38. The further rotational axis 94 runs in this embodiment perpendicular to the longitudinal axis 22 of the pedicle screw 14. However, other angles distinct from 0° or 180° may be envisaged as well.

This additional rotational degree of freedom further increases the range of positions at which the rod 12a can be fixed to the pedicle screw 14. Among others, the polyaxial adjustability of the tulip 20 includes the capability of tilting the tulip 20 around a tilt axis which runs parallel to the further rotational axis 94. Such a pair of parallel rotational axes can be used to adjust the height of the rod 12a received in the tulip 20 with respect to the pedicle screw 14. For example, if the protrusion portion 40' in FIG. 9 is rotated towards the viewer and the tulip 20 is tilted backward by the same angle, the rod 12a will not change its orientation, but will be positioned lower, i. e. closer to the pedicle screw 14, than before.

For fixing the protrusion portion 40' at a certain rotational position, the second fixing screw 86 which fixes the cylindrical portion 38 of the head member 32 to the head 30 of the pedicle screw 14 (see FIG. 7) may be used. For example, the head of the second fixing screw 86 may directly press on a shaft of the joint 92 so that the shaft cannot rotate anymore.

### b) Fixing rod and tulip simultaneously

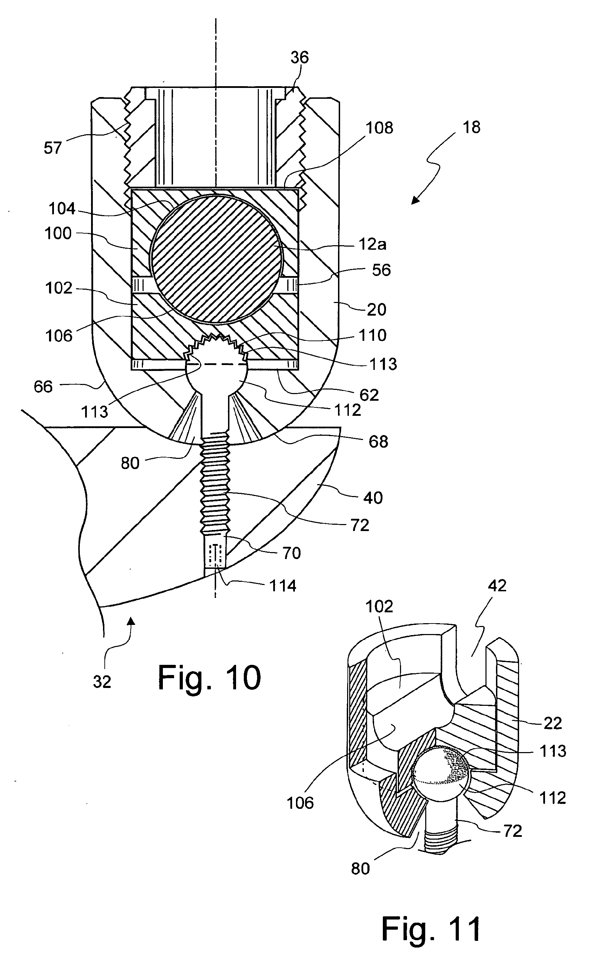

FIGS. 10 to 12 show another embodiment of a connector 18 in accordance with the present invention in a cross-section similar to FIGS. 5a and 5b, in a perspective partial section and in an exploded view, respectively.

The connector 18 is configured such that the tulip 20 can be fixed with respect to the projection 40 of the head member 32 with the rod 12a in place. More particularly, the tulip 20 is fixed not with the help of the first fixing screw 72, but using the clamp screw 36. The clamp screw 36 thus fixes simultaneously the rod 12a to the tulip 20 and the tulip 20 to the projection 40 of the head member 32.

To this end the tulip 20 of this embodiment has a cylindrical bore 56 which is configured to receive an upper clamp block 100 and a lower clamp block 102 whose shape can best be seen in the exploded view of FIG. 12. Both clamp blocks 100, 102 have a concave cylindrical recess 104 and 106, respectively, which has approximately the same radius as the rod 12a. The upper clamp block 100 has, opposite to the cylindrical recess 104, an abutment face 108 on which the clamp screw 36 rests when it is screwed into the tulip 20. The lower clamp block 102 has on its side opposite the cylindrical recess 106 a spherical recess 110 having a corrugated or otherwise roughened surface 111.

The first fixing screw 72 is provided in this embodiment with a ball head 112 which has approximately the same radius of curvature as the spherical recess 110 and which is also provided with a corrugated or otherwise roughened surface 113. This surface 113 increases the friction between the ball head 112 and the spherical recess 110. For tightening the first fixing screw 72 in the threaded bore 70 of the projection 40, the opposite end of the first fixing screw 72 is provided with a socket 114 into which a suitable driver can be inserted.

As long as the clamp screw 36 is not tightly screwed into the thread 57 of the tulip 20, the upper clamp block 100 does not rest firmly on the rod 12a. Then also the lower clamp block 102 is not significantly loaded, and consequently the friction between the surfaces 111, 113 of the recess 110 and the ball head 112 is small enough to adjust the tulip 20 on the projection 40 polyaxially.

Once the optimum position of the tulip 20 on the projection 40 has been found, the clamp screw 36 will be tightened. Then the upper clamp block 100 presses down the rod 12a, which in turn presses down the lower clamp block 102 on the ball head 112. The friction between the spherical recess 110 and the ball head 112 is now, due to the considerable load applied by the clamp screw 36, so large that the tulip 20 cannot be adjusted anymore. The pressure exerted by the clamp screw 36 thus not only fixes the rod 12a in the tulip 20, but also the tulip 20 to the projection 40 of the head member 32.

### c) Fixing rod, tulip and head member simultaneously

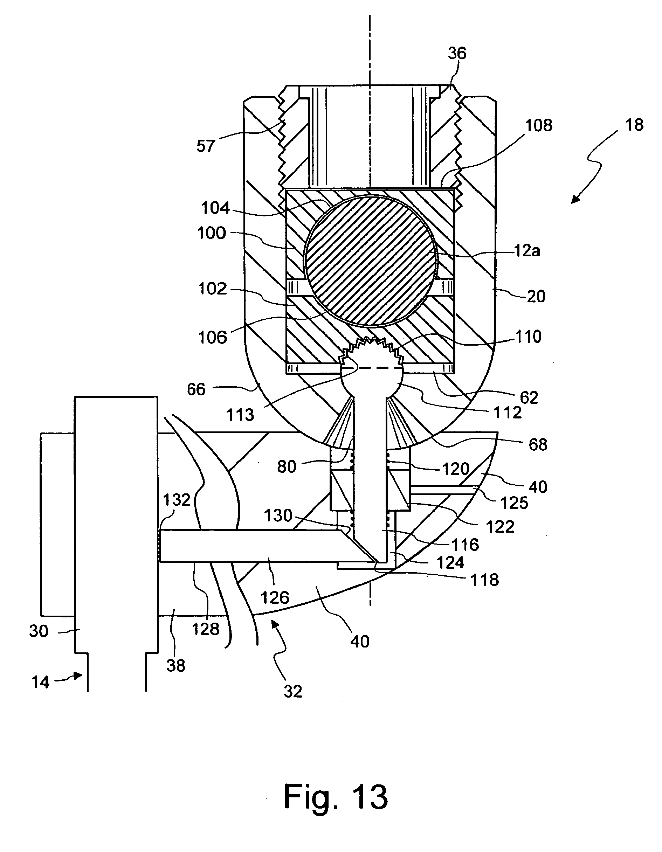

FIG. 13 shows a connector 18 in a cross-section similar to FIG. 10 according to a still further embodiment. In this embodiment tightening of the clamp screw 36 does not only fix the rod 12a in the tulip 20 and simultaneously the tulip 20 to the projection 40 of the head member 32, but also the entire head member 32 to the head 30 of the pedicle screw 14.

To this end the first fixing screw 72 is replaced by a pin 116 which bears on one side the same ball head 112 as shown in FIG. 10. On its opposite side the pin 116 is provided with an oblique abutment face 118. A middle section of the pin 116 is provided with circumferential ridges 120. The ridges 120 interact with a one-way clutch 122 that is inserted into a bore 124 extending into the projection 40. Thus the pin 116 can be inserted into the bore 124, but cannot be removed from the bore 124 without opening the one-way clutch 122 with the help of a suitable instrument that is inserted into an access channel 125.

The pin 116 interacts with a second pin 126 which is arranged perpendicularly to the first pin 116. Also the second pin 126 is received in a bore 128 provided in the projection 40. At one end the second pin 126 is provided with an oblique abutment face 130 and on the other end with a corrugated or otherwise roughened concave friction face 132.

If the clamp screw 36 is tightened, the first pin 116 is pressed through the bore 124 until its oblique abutment face 118 rests on the oblique abutment face 130 of the second pin 126. Upon further tightening the clamp screw 36, the first pin 116 will urge the second pin 126 sideward so that its friction face 132 presses against the cylindrical head 30 of the pedicle screw 14. This clamp mechanism thus ensures that by tightening the clamp screw 36 not only the rod 12a is fixed between the upper and lower clamp block 100, 102, but also that the tulip 22 is fixed with respect to the head member 32 of the connector 18. Furthermore, the clamping mechanism ensures, via the leverage provided by the two pins 116, 126, that the force exerted by the clamp screw 36 is guided towards the friction face 132 which presses against the head 30 of the pedicle screw 14 so that the head member 32 cannot rotate anymore around the pedicle screw 14.

## Claims

1. A spine fixation system (10), comprising:
a) a rod (12a, 12b) which is configured to extend over a portion of the spine,
b) a plurality of fasteners (14), wherein each fastener has a longitudinal axis (22) and is configured to be secured to a vertebra (V1, V2, V3) to be treated,
c) a plurality of connectors (18), wherein each connector
- is connected to, or is capable of being connected to, one of the fasteners (14),
- has a seat member (20) for receiving and fixing the rod (12a, 12b), wherein the seat member (20) is capable of being fixed in different rotational positions with regard to a rotational axis (46) that extends, or is capable of being positioned such that it extends, parallel to but not coinciding with the longitudinal axis (22) of the fastener (14) to which the respective connector (18) is connected,
- is configured such that the rod (12a, 12b) is allowed, when received in the seat member (20), to swivel over the fastener (14) when the seat member (20) rotates around the rotational axis (46), and
- comprises a head member (32) which is connected to, or is capable of being connected to, one of the fasteners (14), and which supports the seat member (20) so as to enable at least rotational movement of the seat member (20) with regard to the head member (32) before the position of the seat member (20) is fixed, wherein the head member (32) is capable of being fixed to the fastener (14), to which the respective connector (18) is connected, in different rotational positions with regard to the longitudinal axis (22) of this fastener (14).

2. The system of claim 1, wherein each fastener (14) is configured to be secured to a pedicle (16) of the vertebra (V1, V2, V3) to be treated.

3. The system of claim 1 or 2, wherein the fasteners (14) are screws.

4. The system of any of the preceding claims, wherein the rotational axis (46) is, for enabling the seat member (20) to be polyaxially adjusted, capable of being fixed in different tilting positions at least within a cone (48) of tilting angles.

5. The system of claim 4, wherein the cone (48) has an axis of symmetry which extends parallel to, but does not coincide with, the longitudinal axis (22) of the fastener (14) to which the respective connector (18) is connected.

6. The system of any of the preceding claims, wherein each connector (18) is configured to be connected to the respective fastener (14) after the fastener has been secured to the vertebra (V1, V2, V3) to be treated.

7. The system of any of the preceding claims, wherein the seat member (20) has, in its cross-section, a U-shaped recess (42) for receiving the rod (12a, 12b).

8. The system of claim 7, wherein the recess (42) is configured such that the lowest portion of the rod (12a, 12b), if received in the recess (42), is arranged, along a direction parallel to the longitudinal axis (22), higher than the highest portion (54) of the head member (32).

9. The system of any of the preceding claims, wherein the connector comprises a clamp mechanism (36) for fixing the rod (12a, 12b) to the seat member (42).

10. The system of claim 9, wherein the clamp mechanism (36, 100, 102, 110, 111, 112, 113) is configured such that operation of the clamp mechanism by a user fixes the rod (12a) to the seat member (20) and simultaneously fixes the seat member (20) to the head member (32).

11. The system of claim 10, wherein the clamp mechanism (36, 100, 102, 110, 111, 112, 113, 116, 118, 120, 122, 126, 128) is configured such that operation of the clamp mechanism by a user fixes the rod (12a) to the seat member (20) and simultaneously fixes the seat member (20) to the head member (32) and also fixes the head member (32) to the fastener (14).

12. The system of any of the preceding claims, wherein the head member (32) comprises a first head portion (38), which is fixed to the fastener (14) or is capable of being fixed to it, and a second head portion (40'), which supports the seat member (20), wherein the second head portion (40') is capable of being fixed to the first head portion (38) in different rotational positions with regard to a further rotational axis (94) that is arranged non-parallel to the longitudinal axis (22) of the fastener (14).

13. The system of claim 12, wherein the further rotational axis (94) is arranged perpendicularly to the longitudinal axis (22) of the fastener (14).

## Patentansprüche

1. Ein Wirbelsäulenfixierungssystem (10), das umfasst:
a) einen Stab (12a, 12b), welcher dazu ausgebildet ist, sich über einen Bereich der Wirbelsäule zu erstrecken;
b) eine Vielzahl an Befestigungsmitteln (14), wobei jedes Befestigungsmittel eine longitudinale Achse (22) aufweist und dazu ausgebildet ist, an einem zu behandelnden Wirbelknochen (V1, V2, V3) befestigt zu werden,
c) eine Vielzahl an Verbindungsgliedern (18), wobei jedes Verbindungsglied
- mit einem der Befestigungsmittel (14) verbunden oder verbindbar ist ,
- ein Sitzelement (20) zum Aufnehmen und Befestigen des Stabs (12a, 12b) aufweist, wobei das Sitzelement (20) in verschiedenen Rotationsstellungen in Bezug auf eine Rotationsachse (46) befestigbar ist, die sich parallel zu, aber nicht zusammenfallend mit der longitudinalen Achse (22) des Befestigungsmittels (14), mit dem das entsprechende Verbindungsglied (18) verbunden ist, erstreckt oder derart positionierbar ist, dass sie sich erstreckt,
- derart ausgebildet ist, dass der Stab (12a, 12b), wenn dieser im Sitzelement (20) aufgenommen ist, über das Befestigungsmittel (14) hinweg schwenkbar ist, wenn das Sitzelement (20) um die Rotationsachse (46) rotiert, und
- ein Kopfelement (32) aufweist, das mit einem der Befestigungsmittel (14) verbunden oder verbindbar ist und welches das Sitzelement (20) trägt, um zumindest eine Rotationsbewegung des Sitzelements (20) in Bezug auf das Kopfelement (32) zu erlauben, bevor die Position des Sitzelements (20) festgelegt ist, wobei das Kopfelement (32) an dem Befestigungsmittel (14), mit dem das entsprechende Verbindungsglied (18) verbunden ist, in verschiedenen Rotationsstellungen bezüglich der longitudinale Achse (22) dieses Befestigungsmittels (14) befestigbar ist.

2. Das System nach Anspruch 1, wobei jedes Befestigungsmittel (14) dazu ausgebildet ist, an einem Pedikel (16) des zu behandelnden Wirbelknochens (V1, V2, V3) befestigt zu werden.

3. Das System nach Anspruch 1 oder 2, wobei die Befestigungsmittel (14) Schrauben sind.

4. Das System nach einem der vorhergehenden Ansprüche, wobei die Rotationsachse (46), um zu ermöglichen, dass das Sitzelement (20) polyaxial eingestellt werden kann, in verschiedenen Neigungspositionen zumindest innerhalb eines Kegels (48) von Neigungswinkeln befestigbar ist.

5. Das System nach Anspruch 4, wobei der Kegel (48) eine Symmetrieachse aufweist, die sich parallel zu der longitudinalen Achse (22) des Befestigungsmittels (14) erstreckt, mit dem das entsprechende Verbindungsglied (18) verbunden ist, aber mit ihr nicht zusammenfällt.

6. Das System nach einem der vorhergehenden Ansprüche, wobei jedes Verbindungsglied (18) dazu ausgebildet ist, mit dem entsprechenden Befestigungsmittel (14) verbunden zu werden, nachdem das Befestigungsmittel an dem zu behandelnden Wirbelknochen (V1, V2, V3) befestigt worden ist.

7. Das System nach einem der vorhergehenden Ansprüche, wobei das Sitzelement (20) in seinem Querschnitt eine U-förmige Aussparung (42) zum Aufnehmen des Stabs (12a, 12b) aufweist.

8. Das System nach Anspruch 7, wobei die Aussparung (42) derart ausgebildet ist, dass der unterste Abschnitt des Stabs (12a, 12b), falls dieser in der Aussparung (42) aufgenommen ist, entlang einer Richtung, die parallel zu der longitudinalen Achse (22) ist, oberhalb des obersten Abschnitts (54) des Kopfelements (32) angeordnet ist.

9. Das System nach einem der vorhergehenden Ansprüche, wobei das Verbindungsglied einen Klemmmechanismus (36) zum Befestigen des Stabs (12a, 12b) an dem Sitzelement (42) aufweist.

10. Das System nach Anspruch 9, wobei der Klemmmechanismus (36, 100, 102, 110, 111, 112, 113) derart ausgebildet ist, dass eine Betätigung des Klemmmechanismus durch einen Benutzer den Stab (12a) am Sitzelement (20) befestigt und gleichzeitig das Sitzelement (20) am Kopfelement (32) befestigt.

11. Das System nach Anspruch 10, wobei der Klemmmechanismus (36, 100, 102, 110, 111, 112, 113, 116, 118, 120, 122, 126, 128) derart ausgebildet ist, dass eine Betätigung des Klemmmechanismus durch einen Benutzer den Stab (12a) am Sitzelement (20) befestigt und gleichzeitig das Sitzelement (20) an dem Kopfelement (32) befestigt und auch das Kopfelement (32) am Befestigungsmittel (14) befestigt.

12. Das System nach einem der vorhergehenden Ansprüche, wobei das Kopfelement (32) zunächst einen ersten Kopfabschnitt (38), der am Befestigungselement (14) befestigt oder daran befestigbar ist, und einen zweiten Kopfabschnitt umfasst (40'), der das Sitzelement (20) trägt, wobei der zweite Kopfabschnitt (40') an dem ersten Kopfabschnitt (38) in verschiedenen Rotationsstellungen bezüglich einer weiteren Rotationsachse (94) befestigbar ist, die nicht parallel zur longitudinalen Achse (22) des Befestigungsmittels (14) angeordnet ist.

13. Das System nach Anspruch 12, wobei die weitere Rotationsachse (94) senkrecht zur longitudinalen Achse (22) des Befestigungsmittels (14) angeordnet ist.

## Revendications

1. Système de fixation de colonne vertébrale (10), comprenant :
a) une tige (12a, 12b), laquelle est configurée de manière à s'étendre sur une partie de la colonne vertébrale,
b) une pluralité de moyens d'ancrage (14), où chaque moyen d'ancrage comporte un axe longitudinal (22) et est configuré de manière à être fixé sur une vertèbre (V1, V2, V3) devant être traitée,
c) une pluralité de raccords (18), où chaque raccord
- est relié, ou peut être relié, à l'un des moyens d'ancrage (14),
- comporte un élément de siège (20) destiné à recevoir et à fixer la tige (12a, 12b), où l'élément de siège (20) peut être fixé dans différentes positions rotationnelles par rapport à un axe de rotation (46) qui s'étend, ou peut être positionné de telle sorte qu'il s'étende, parallèlement à l'axe longitudinal (22), mais sans coïncider avec ce dernier, du moyen d'ancrage (14) auquel le raccord (18) respectif est relié,
- est configuré de telle sorte que la tige (12a, 12b) est libre, lorsqu'elle est reçue dans l'élément de siège (20), de pivoter par rapport au moyen d'ancrage (14) lorsque l'élément de siège (20) tourne autour de l'axe de rotation (46), et
- comprend un élément de tête (32), lequel est relié, ou peut être relié, à l'un des moyens d'ancrage (14), et lequel supporte l'élément de siège (20) de sorte à permettre au moins un mouvement rotationnel de l'élément de siège (20) par rapport à l'élément de tête (32) avant que la position de l'élément de siège (20) ne soit fixée, où l'élément de tête (32) peut être fixé sur le moyen d'ancrage (14), auquel le raccord (18) respectif est relié, dans différentes positions rotationnelles par rapport à l'axe longitudinal (22) de ce moyen d'ancrage (14).

2. Système selon la revendication 1, dans lequel chaque moyen d'ancrage (14) est configuré de manière à être fixé sur un pédicule (16) de la vertèbre (V1, V2, V3) devant être traitée.

3. Système selon la revendication 1 ou 2, dans lequel les moyens d'ancrage (14) sont des vis.

4. Système selon l'une quelconque des revendications précédentes, dans lequel l'axe de rotation (46) peut, afin de permettre à l'élément de siège (20) d'être ajusté de manière poly-axiale, être fixé dans différentes positions d'inclinaison au moins à l'intérieur d'un cône (48) d'angles d'inclinaison.

5. Système selon la revendication 4, dans lequel le cône (48) présente un axe de symétrie, lequel s'étend parallèlement à l'axe longitudinal (22), mais sans coïncider avec ce dernier, du moyen d'ancrage (14) auquel le raccord (18) respectif est relié.

6. Système selon l'une quelconque des revendications précédentes, dans lequel chaque raccord (18) est configuré de manière à être relié au moyen d'ancrage (14) respectif après que le moyen d'ancrage a été fixé sur la vertèbre (V1, V2, V3) devant être traitée.

7. Système selon l'une quelconque des revendications précédentes, dans lequel l'élément de siège (20) comporte, dans sa section transversale, un évidement en forme de U (42) destiné à recevoir la tige (12a, 12b).

8. Système selon la revendication 7, dans lequel l'évidement (42) est configuré de telle sorte que la partie la plus basse de la tige (12a, 12b), si elle est reçue dans l'évidement (42), est agencée, le long d'une direction parallèle à l'axe longitudinal (22), plus haut que la partie la plus haute (54) de l'élément de tête (32).

9. Système selon l'une quelconque des revendications précédentes, dans lequel le raccord comprend un mécanisme de serrage (36) destiné à fixer la tige (12a, 12b) sur l'élément de siège (42).

10. Système selon la revendication 9, dans lequel le mécanisme de serrage (36, 100, 102, 110, 111, 112, 113) est configuré de telle sorte qu'une mise en oeuvre du mécanisme de serrage par un utilisateur fixe la tige (12a) sur l'élément de siège (20) et fixe simultanément l'élément de siège (20) sur l'élément de tête (32).

11. Système selon la revendication 10, dans lequel le mécanisme de serrage (36, 100, 102, 110, 111, 112, 113, 116, 118, 120, 122, 126, 128) est configuré de telle sorte qu'une mise en oeuvre du mécanisme de serrage par un utilisateur fixe la tige (12a) sur l'élément de siège (20) et fixe simultanément l'élément de siège (20) sur l'élément de tête (32) et fixe également l'élément de tête (32) sur le moyen d'ancrage (14).

12. Système selon l'une quelconque des revendications précédentes, dans lequel l'élément de tête (32) comprend une première partie de tête (38), laquelle est fixée sur le moyen d'ancrage (14) ou peut être fixé sur celui-ci, et une deuxième partie de tête (40'), laquelle supporte l'élément de siège (20), où la deuxième partie de tête (40') peut être fixée sur la première partie de tête (38) dans différentes positions rotationnelles par rapport à un axe de rotation supplémentaire (94) qui est agencé de manière non parallèle par rapport à l'axe longitudinal (22) du moyen d'ancrage (14).

13. Système selon la revendication 12, dans lequel l'axe de rotation supplémentaire (94) est agencé perpendiculairement à l'axe longitudinal (22) du moyen d'ancrage (14).
